# FASCICULE DE BREVET EUROPEEN

(11) **EP 1 588 694 B1**
(45) Date de publication et mention de la délivrance du brevet: **23.05.2007**
(21) Numéro de dépôt: 05290626.0
(22) Date de dépôt: 22.03.2005
(51) Int. Cl.: A61K 8/02, A61Q 19/00, A61Q 19/08, A61Q 5/00

(54) **Kit de formulation d'un produit cosmétique**
Kosmetisches Mittel Formulierungskit
Cosmetic product formulation kit

(30) Priorité: 19.04.2004 FR 0404125
(43) Date de publication de la demande: 26.10.2005
(73) Titulaire: L'ORÉAL, 75008 Paris (FR)
(72) Inventeur: Legendre, Jean-Yves, 75015 Paris (FR); Guiramand, Carole, 78350 Jouy en Josas (FR); Cassin, Guillaume, 91140 Villebon sur Yvette (FR); Simonnet, Jean-Thierry, 94240 Cachan (FR)
(74) Mandataire: Rasson, Catherine

(56) Documents cités:
- WO-A-02/05789
- FR-A- 2 840 221
- US-A1- 2003 186 826

## Description

La présente invention a trait à des kits de formulation de produits cosmétiques, et en particulier à un kit de formulation de produits cosmétiques comportant au moins un film fin anhydre et une composition aqueuse, le film et la composition étant destinés à être mélangés extemporanément pour former un produit cosmétique qui peut être notamment un produit de maquillage ou de soin de la peau, des muqueuses, des phanères ou des cheveux.

L'invention se rapporte aussi à une utilisation cosmétique de ce produit pour le traitement cosmétique de la peau, des muqueuses, des phanères ou des cheveux.

L'invention se rapporte également à un procédé de formulation de produit à partir d'un kit.

Les produits cosmétiques contiennent différents composés qui jouent chacun un rôle, soit pour la galénique du produit soit pour l'activité du produit soit pour toute autre propriété du produit comme par exemple son odeur ou sa couleur. C'est ainsi par exemple que la présence des actifs contribue à l'efficacité du produit sur la peau, par exemple pour maintenir la peau en bonne santé et/ou à améliorer l'état de la peau, tandis que les agents de viscosité permettent de régler la texture du produit et que les colorants lui confèrent une teinte déterminée. En outre, le plus souvent, ces produits contiennent un grand nombre de composés différents, et il peut arriver qu'il ne soit pas souhaitable, pour diverses raisons, que tous les composés soient mélangés avant utilisation du produit. Par exemple, des composés et notamment des actifs peuvent être incompatibles entre eux ou entraîner une instabilité de la composition les contenant.

Par ailleurs, les utilisateurs souhaitent de plus en plus disposer d'une composition adaptée à l'usage qu'ils veulent en faire au moment où ils en disposent. Ainsi, selon l'humeur, les saisons, la température extérieure, l'utilisateur aspire à disposer d'une composition adaptée à son besoin du moment, par exemple disposer d'une composition plus ou moins épaisse ou plus ou moins concentrée en actif ou ayant une coloration ou un parfum particulier, ces particularités pouvant varier dans le temps.

Aussi, le kit faisant l'objet de l'invention permet de résoudre ces problèmes. Ce kit a plusieurs formes de réalisation.

Ainsi, l'invention a pour objet un kit de formulation d'un produit cosmétique comprenant (1) une composition aqueuse contenant au moins un corps gras, et (2) au moins un film anhydre hydrosoluble comportant au moins un composé qui est apte à modifier la composition aqueuse.

La composition aqueuse et le ou les films sont mélangés extemporanément en vue de former ledit produit cosmétique.

Ce produit cosmétique peut être utilisé notamment comme produit de soin ou de maquillage de la peau, des muqueuses, des phanères ou des cheveux:

On entend dans la présente demande par « composé qui est apte à modifier la composition aqueuse » tout composé qui va modifier l'activité et/ou l'aspect de la dite composition, par exemple son activité par ajout d'un ou plusieurs actifs, ou son aspect par ajout d'un épaississant (modification de la viscosité) ou par ajout d'un colorant (modification de la couleur) ou par ajout d'un parfum (modification de l'odeur).

On entend par "corps gras" tout composé insoluble dans l'eau, ce composé pouvant être une huile (corps gras liquide) ou un corps gras solide. Il s'agit de préférence d'une huile.

Le mélange extemporané est obtenu par simple mélange des quantités voulues de la composition aqueuse et du ou des films, ces quantités étant déterminées selon le but final recherché. Le mélange obtenu constitue un produit cosmétique à usage unique. Toutefois, le kit peut contenir plusieurs films de compositions identiques ou différentes, ce qui permet d'obtenir plusieurs fois un produit cosmétique identique ou différent à usage unique.

L'invention a aussi pour objet un kit de formulation d'un produit cosmétique comprenant :
i) une composition aqueuse ; et
ii) au moins un film anhydre hydrosoluble comportant au moins un composé qui, pour des raisons de stabilité, ne peut être mélangé à la composition que de façon extemporanée en vue de former ledit produit cosmétique.

L'invention a aussi pour objet un kit de formulation d'un produit cosmétique comprenant :
i) une composition aqueuse ;
ii) au moins un premier film anhydre hydrosoluble ; et
iii) au moins un second film anhydre hydrosoluble, le second film étant distinct du premier, notamment par la concentration et/ou par la nature d'au moins un composé qu'il contient.

Ledit produit cosmétique est obtenu en ajoutant à la composition aqueuse, un ou plusieurs desdits premiers films et un ou plusieurs desdits seconds films.

L'invention a encore pour objet un kit de formulation customisée d'un produit cosmétique, notamment de soin ou de maquillage, comprenant :
i) une composition aqueuse ;
ii) une pluralité de films anhydres hydrosolubles, identiques ou différents, destiné(s) à être mélangé(s) à la composition aqueuse pour former ledit produit cosmétique, et
iii) des instructions, notamment sur une notice explicative, pour formuler à façon ledit produit cosmétique en fonction du nombre de films anhydres hydrosolubles identiques ou différents à mélanger à la composition.

On entend par "formulation customisée", une formulation adaptée à la demande du consommateur au moment de l'utilisation.

L'invention a aussi pour objet un procédé de formulation d'un produit cosmétique, notamment de soin ou de maquillage, consistant à :
a) déterminer au moins une caractéristique du produit cosmétique à formuler, notamment une teinte ou une concentration , caractéristique due à au moins un composé présent soit dans une pluralité de films anhydres hydrosolubles identiques, soit dans une pluralité de films anhydres hydrosolubles différant les uns des autres notamment par la concentration et/ou la nature d'au moins un composé qu'ils contiennent ; et
b) ajouter à une composition aqueuse, un nombre desdits films identiques ou différents, ledit nombre étant fonction de ladite caractéristique du produit cosmétique à formuler.

L'invention a aussi pour objet un procédé de modification des caractéristiques de rhéologie, de couleur et/ou de parfum d'une composition aqueuse en vue de la formulation d'un produit cosmétique, notamment de soin ou de maquillage, consistant à ajouter à une composition aqueuse, un ou plusieurs films anhydres hydrosolubles contenant un ou plusieurs composés aptes à modifier la rhéologie, la couleur et/ou le parfum d'une composition aqueuse.

Certes, il est connu d'utiliser des films anhydres à dissolution immédiate au contact de l'eau ou de la salive, et des films comestibles visant à envelopper les aliments afin d'accroître leur durée de conservation, et cette technique a fait l'objet de demandes de brevets (par exemple US-A-5,965,708, US-A-5,962,053, JP-A-10/215792). De même, ce type de technologie est utilisé dans le domaine de la pharmacie pour administrer des principes actifs par voie orale sous forme de formulations à délitement buccal instantané (voir par exemple les documents WO-A-2002/085119, WO-A-2002/043657, WO-A-2001/070194), ou par application sur d'autres muqueuses, comme le vagin (EP-A-1,110,546) et sur les plaies (JP-A-63/220876). En outre, le document JP-A-2002/212027 décrit l'obtention et la composition de préparations cosmétiques sous forme de films hydrosolubles, et les documents WO-A-2002/05789, US-A-2002/0127254 et WO-A-2003/075812 décrivent l'obtention et l'administration de films polymériques anhydres pour une administration directe de compositions cosmétiques sur la peau préalablement mouillée. De plus, le document US-A-2003/0186826 décrit une composition cosmétique sèche à base de polymères et de tensioactifs, à administrer sur la peau ou les cheveux avec de l'eau. Toutefois, ces documents n'envisagent jamais la préparation extemporanée d'un produit cosmétique ou dermatologique à partir de kits comportant des films fins et une composition aqueuse, notamment une composition aqueuse sous forme d'émulsion.

Certes, il est connu par le document FR-A-2,840,221, des films contenant des actifs destinés à être libérés dans un liquide, ce liquide pouvant être de l'eau ou un solvant tel que l'huile ou l'éthanol. Toutefois, il n'est jamais envisagé dans ce document d'introduire des films dans un milieu complexe tel qu'une émulsion, ou de disposer de kits permettant d'obtenir un produit adapté au moment de l'utilisation.

Il n'a donc jamais été décrit dans l'art antérieur, un kit cosmétique permettant d'obtenir des produits contenant des composés incompatibles, et permettant par ailleurs à l'utilisateur d'obtenir un produit adapté à son type de peau ou au but recherché au moment de l'utilisation, aussi bien en ce qui concerne les actifs contenus qu'en ce qui concerne la texture du produit ou toute autre spécificité du produit pour qu'il soit adapté au besoin de l'utilisateur au moment de l'utilisation, ce qui peut se traduire par un parfum ou un colorant particulier ou par des effets optiques spécifiques ou par un indice de protection solaire déterminé ou un pH particulier ou toute autre caractéristique qui peut varier selon l'envie de l'utilisateur au moment où il utilise le kit. La présente invention met à disposition un tel kit.

Le kit selon l'invention qui combine l'utilisation d'au moins un film anhydre hydrosoluble à base de polymères et d'une composition aqueuse, notamment sous forme d'émulsion, permet de moduler extemporanément par l'utilisateur, la composition, la rhéologie, la couleur ou la concentration en actif du produit final. En outre, dans le cas de l'application d'actifs peu stables en milieu liquide ou semi-solide, leur incorporation dans le ou les films anhydres à dissoudre extemporanément permet de garantir leur efficacité. De même, une bonne stabilité colloïdale du produit final peut être obtenue par une dissolution extemporanée d'un excipient dont les performances n'auraient pu être maintenues en cours du temps en milieu liquide.

Le produit cosmétique final est obtenu par mélange extemporané d'un ou plusieurs films anhydres hydrosolubles et d'une quantité appropriée de la composition aqueuse. On entend par "quantité appropriée" de composition aqueuse, une quantité telle que le ou les films s'y dissolvent rapidement. Cette quantité peut aller par exemple de 10 à 1000 mg, de préférence de 50 à 800 mg et mieux de 100 à 500 mg. La dose appropriée de composition aqueuse peut être obtenue en utilisant des formes de présentation en mono-dose, telles que des sachets, des tubes, des ampoules, des seringues pré-remplies, des capsules molles, des coques ou barquettes en plastique thermoformé. Une dose appropriée peut également être obtenue à partir d'une présentation multi-doses en utilisant un système distribuant une dose pré-définie. Un tel système peut être un flacon pompe, un aérosol, une pipette ou une seringue graduée, un compte-gouttes.

D'autres objets encore apparaîtront dans la description détaillée qui suit.

### A. Films anhydres hydrosolubles

Par "film", on entend dans la présente demande, un solide fin et préhensible. On entend par "fin", un solide ayant une épaisseur d'au maximum 1000 µm. Ce film a généralement une dimension adéquate pour pouvoir être facilement manipulé par l'utilisateur. Il peut avoir une forme de carré, de rectangle, de disque ou toute autre forme. Chaque film a généralement une épaisseur de 10 µm à 1000 µm, de préférence de 20 à 500 µm et mieux de 50 à 300 µm. Il peut avoir une surface de 0,25 à 25 cm² et de préférence de 2 à 10 cm².

Par ailleurs, par "film anhydre", on entend dans la présente demande, un film contenant moins de 10 % en poids d'eau, de préférence moins de 5 % en poids par rapport au poids total du film, et de préférence encore, ne contenant pas d'eau.

En outre, on entend dans la présente demande par « film hydrosoluble », un film qui se dissout dans l'eau. Il s'agit d'un film constitué d'un ou de plusieurs polymères hydrosolubles ou hydrodispersibles. On entend par "hydrosolubles ou hydrodispersibles" des polymères ayant une solubilité dans l'eau mesurée à 25°C au moins égale à 0,1 gramme/litre (g/L) (obtention d'une solution macroscopiquement isotrope et transparente, colorée ou non). Cette solubilité est de préférence supérieure ou égale à 1 g/L. Les polymères pour constituer ces films peuvent être d'origines synthétique ou naturelle, et être, le cas échéant, modifiés par réactions chimiques. Ils peuvent être ou non filmogènes. Ces polymères doivent être physiologiquement acceptables c'est-à-dire compatible avec la peau, les muqueuses, les cheveux et le cuir chevelu.

Ces polymères hydrosolubles ou hydrodispersibles peuvent par exemple être choisis parmi (1) les polymères de type protéique, tels que les protéines de blé ou de soja ; la kératine, par exemple les hydrolysats de kératine et les kératines sulfoniques ; la caséine ; l'albumine ; le collagène ; la gluteline ; le glucagon ; le gluten ; la zéine ; les gélatines et leurs dérivés ; (2) les polymères dérivant de la chitine ou du chitosane, tels que les polymères anioniques, cationiques, amphotères ou non ioniques de chitine ou de chitosane ; (3) les polymères polysaccharidiques tels que notamment (i) les polymères cellulosiques, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose ; et (ii) les amidons et leurs dérivés ; (4) les polymères ou copolymères acryliques tels que les polyacrylates, les polyméthacrylates et leurs copolymères ; (5) les polymères vinyliques tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques ; (6) les polymères d'origine naturelle, éventuellement modifiés, tels que la gomme arabique, la gomme de guar, les dérivés du xanthane, la gomme de karaya ; les alginates, les carraghénanes, les ulvanes et autres colloïdes algaux ; les glycoaminoglycanes, l'acide hyaluronique et ses dérivés ; la gomme laque, la gomme sandaraque, les dammars, l'élémis, les copals ; l'acide désoxyribonucléique; les mucopolysaccharides, tels que l'acide hyaluronique, le sulfate de chondroïtine ; et les mélanges de ces polymères.

On peut encore citer comme polymères hydrosolubles, les caprolactames, la pullulane, la pectine, la mannane et les galactomannanes, les glucomannanes et leurs dérivés.

Bien entendu, les films conformes à l'invention peuvent comprendre un ou plusieurs de ces polymères.

La quantité de polymère(s) hydrosoluble(s) dans le film anhydre va généralement de 5 à 100 % en poids, en particulier de 10 à 95 % en poids, et plus particulièrement de 20 à 90 % en poids par rapport au poids total dudit film.

Les films peuvent être conditionnés dans un article facilitant leur préhension, tel que celui décrit dans la demande de brevet FR0351002 dont le contenu est inclus dans la présente demande par référence. Les films peuvent notamment être conditionnés dans une boîte plastique distributrice, dans un sachet individuel ou dans un blister, contenus dans le kit de l'invention. Les films peuvent être conditionnés dans un boîtier de type à tiroir ou à couvercle articulé sur un fond, le dit boîtier pouvant comporter des moyens destinés à faciliter la distribution des articles. Les moyens de distribution peuvent être du type de ceux décrits par exemple dans les documents US-A-2,973,882, GB-A-2,358,627, CH-A-461025, ou US-A-6,578,732.

Outre le ou les polymères hydrosolubles, le film peut comprendre un ou plusieurs plastifiants, par exemple choisis parmi la glycérine, le sorbitol, les mono- et/ou disaccharides, le dipropylène glycol, le butylène glycol, le pentylène glycol, les polyéthylène glycols tels que le PEG-400. La quantité de plastifiant(s) peut aller par exemple de 1 à 40 % en poids et mieux de 2 à 15 % en poids par rapport au poids total du film.

Conformément à l'invention, le film comprend au moins un composé, notamment un composé apte à modifier la composition aqueuse. On entend par "composé apte à modifier la composition aqueuse" tout composé qui va, par mélange du film avec la composition aqueuse, modifier la dite composition et par exemple modifier la rhéologie, la texture, l'activité, la couleur, le parfum ou le pH de la dite composition. Ce composé peut être simplement le polymère hydrosoluble présent dans le film, qui, de par sa nature, va modifier la rhéologie de la composition aqueuse et lui donner une texture nouvelle, ou ce peut être un ou plusieurs composés choisis parmi les actifs ou bien les adjuvants de formulation tels que par exemple les agents de rhéologie, les parfums, les matières colorantes, les tensioactifs, les anti-oxydants, les agents régulateurs de pH. Ces composés seront détaillés ci-après.

### B. Composition aqueuse

On entend par "composition aqueuse" dans la présente demande, une composition contenant au moins de l'eau.

La composition aqueuse, outre l'eau, peut contenir un solvant organique soluble dans l'eau, choisi par exemple parmi les mono-alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol ; l'acétone ; et leurs mélanges.

Il s'agit d'une composition plus ou moins fluide par opposition à une composition solide. On entend par "composition plus ou moins fluide" dans la présente demande, une composition dont on peut mesurer la viscosité, et qui va du liquide au semi-solide (crème ou pâte molle). La viscosité peut aller par exemple de 1 à 20000 mPa.s (1 à 20000 cPoises), de préférence de 1 à 15000 mPa.s, cette viscosité étant mesurée à 25°C à l'aide du Rheomat RM180 de Rheometric Scientific, cet appareil étant équipé d'un mobile différent selon les viscosités.

Cette composition comporte un support physiologiquement acceptable, c'est-à-dire compatible avec la peau, les muqueuses, les cheveux et le cuir chevelu.

Selon un mode préféré de réalisation de l'invention, la composition aqueuse contient, outre l'eau, au moins un corps gras. On entend par "corps gras" tout composé insoluble dans l'eau, ce composé pouvant être une huile (corps gras liquide) ou un corps gras solide. Il s'agit de préférence d'une huile.

La composition aqueuse peut notamment se présenter sous forme d'émulsions, compositions comportant une phase aqueuse et une phase huileuse dispersées l'une dans l'autre, par exemple d'émulsions eau-dans-huile (E/H) ou huile-dans-eau (H/E) ou multiple (E/H/E ou H/E/H), choisies notamment parmi les émulsions classiques ou les émulsions particulières comme par exemple parmi :
- les émulsions H/E à base de globules huileux pourvus d'un enrobage cristal liquide lamellaire, telles que décrites dans les documents EP-A-641557 et EP-A-705593 ;
- Les émulsions H/E sans tensioactif, stabilisées par des polymères anioniques hydrodispersibles, telles que celles décrites dans le document EP-A-864320 ;
- Les émulsions H/E à base de polymères dérivés d'acide 2-acrylamido 2-méthylpropane sulfonique (Polymère AMPS), telles que décrites dans le document EP-A-815844 ;
- Les émulsions H/E stabilisées par des polymères d'AMPS hydrophobes, telles décrites dans les documents EP-A-1,069,142, WO-A-2002/43689, WO-A-2002/44231, WO-A-2002/44271, WO-A-2002/44270, WO-A-2002/43686, WO-A-2002/44267, WO-A-2002/43688, WO-A-2002/43677, WO-A-2002/43687, WO-A-2002/44230 ;
- Les émulsions fluides à base de polymères thermoassociatifs, telles que décrites dans les documents EP-A-1,355,990, EP-A-1,355,625, EP-A-1,307,501, EP-A-1,363,964 ;
- Les émulsions H/E obtenues par la méthode PIT (émulsion obtenue par inversion de phase. PIT = Phase Inversion Temperature), telles que décrites dans les documents WO-A-89/11907, DE-A-4318171, et EP-A-815846 ;
- Les nanoémulsions telles que celles décrites dans les demandes EP-A-728460, EP-A-780114, EP-A-780115, EP-A-879589, EP-A-1,010,413, EP-A-1,010,414, EP-A-1,010,415, EP-A-1,010,416, EP-A-1,013,338, EP-A-1,016,453, EP-A-1,018,363, EP-A-1,020,219, EP-A-1,025,898, EP-A-1,120,102, EP-A-1,120,101, EP-A-1,160,005, EP-A-1,172,077 et EP-A-1,353,629.

La composition aqueuse de l'invention peut être notamment sous forme de lotions (émulsions fluides).

La composition aqueuse peut être aussi sous forme de produits aqueux moussants, tels que décrits par exemple dans les documents EP-A-1,166,747, EP-A-1,172,096, EP-A-1,172,095, EP-A-1,174,122, EP-A-1,277,463, EP-A-1,295,594, FR-A-2,824,262.

Selon un mode particulier de réalisation de l'invention, la composition aqueuse utilisée selon l'invention est une émulsion. La proportion de la phase huileuse de l'émulsion peut aller de 1 à 80 % en poids, et de préférence de 1 à 50 % en poids par rapport au poids total de la composition. Les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant quand ils sont présents, le sont généralement, en une proportion allant de 0,2 à 30 % en poids, de préférence de 0,3 à 20 % en poids et mieux de 0,5 à 15 % en poids par rapport au poids total de la composition. L'émulsion peut en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ; les éthers d'alcools gras oxyalkylénés (oxyéthylénés et/ou oxypropylénés) ; les esters de sucres, alcoxylé ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100, commercialisé sous la dénomination Arlacel 165 par la société Uniqema ; et les mélanges contenant ces émulsionnants, tels que le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations CARBOPOL 1342 et PEMULEN par la société Noveon ; ou les polymères en émulsion tels que celui commercialisé sous la dénomination Sepigel 305 par la société Seppic (nom INCl : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate /isophtalate/1,4-cyclohexane-diméthanol (nom INCl : Diglycol/CHDM/Isophtalates/SIP Copolymer) vendus sous les dénominations Eastman AQ polymer (AQ35S, AQ38S, AQ55S, AQ48 Ultra) par la société Eastman Chemical. On peut aussi préparer des émulsions sans émulsionnants, stabilisées par des particules siliconées ou des particules d'oxyde métallique tels que TiO2 ou autres.

Quand la composition aqueuse est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité, ou encore les triglycérides des acides caprylique/caprique comme ceux vendus par la société Stearineries Dubois ou ceux vendus sous les dénominations Miglyol 810, 812 et 818 par la société Dynamit Nobel ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCl : Isopropyl Lauroyl sarcosinate) commercialisé sous la dénomination Eldew SL 205 par la société Ajinomoto ;
- les hydrocarbures linéaires ou ramifiés, d'origines minérale ou synthétique, tels que les huiles minérales (mélange d'huiles hydrocarbonées dérivées du pétrole ; nom INCl : Mineral oil), les huiles de paraffine, volatiles ou non, et leurs dérivés, la vaseline, les polydécènes, l'isohexadécane, l'isododécane, l'isoparaffine hydrogéné tel que l'huile de Parléam® commercialisée par la société NOF Corporation (nom INCl ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- leurs mélanges.
   La composition aqueuse peut contenir en outre tout composé additif approprié. Elle peut par exemple contenir un ou plusieurs composés choisis parmi les adjuvants de formulation et/ou les actifs différents de ceux présents dans le ou les films.
   Un des intérêts du kit selon l'invention est par exemple de pouvoir obtenir un produit cosmétique contenant des actifs incompatibles, un ou plusieurs actifs compatibles étant présents dans la composition aqueuse et un ou plusieurs actifs différents et incompatibles avec ceux de la composition aqueuse étant présents dans le ou les films anhydres. Le mélange extemporané de la composition aqueuse et du ou des films aboutit alors à un produit contenant des actifs incompatibles, leur incompatibilité ne présentant pas d'inconvénient puisque l'utilisation du produit est immédiate.

### C. Composés

Le film anhydre hydrosoluble contient au moins un composé, notamment un composé apte à modifier la composition aqueuse. Ce composé peut être simplement le polymère constituant le film, ce polymère étant apte à modifier la rhéologie de la composition, ou bien le film peut contenir un ou plusieurs autres composés. Ces composés peuvent être choisis notamment parmi les actifs cosmétiques, les adjuvants de formulation et leurs mélanges. Il est possible d'utiliser aussi plusieurs films, l'un contenant un adjuvant de formulation, l'autre un adjuvant de formulation différent ou un actif, un autre encore pouvant contenir un autre actif et/ou un autre adjuvant de formulation.

On entend par « actifs cosmétiques » tout composé qui va avoir un effet bénéfique sur la matière kératinique sur laquelle est appliqué le produit final.

On entend par « adjuvant de formulation » tout composé apte à modifier l'aspect de la composition aqueuse et par exemple la rhéologie, la texture, la couleur, le parfum, le pH de la composition aqueuse.

Par ailleurs, comme indiqué ci-dessus, la composition aqueuse peut contenir aussi un ou plusieurs composés choisis parmi les actifs cosmétiques, les adjuvants de formulation et leurs mélanges.

Ainsi, ces composés peuvent être incorporés soit dans la composition aqueuse soit dans un ou plusieurs films, ce qui permet de résoudre les éventuels problèmes d'incompatibilité physico-chimique de ces composés, et aussi d'avoir des produits finaux adaptés à la demande de l'utilisateur. Cela permet aussi de pouvoir obtenir des produits ayant des concentrations en actifs plus élevées que les concentrations que l'on pourrait obtenir en utilisant une seule composition.

Comme indiqué ci-dessus, le kit selon l'invention présente l'avantage de permettre la préparation de produits cosmétiques ou dermatologiques contenant des composés incompatibles. Il présente aussi l'avantage de permettre l'obtention de produits cosmétiques ou dermatologiques contenant des composés sensibles à un stimulus extérieur, notamment sensibles à l'eau, à l'oxydation, à la lumière et/ou à l'élévation de la température. Ces composés sensibles à un stimulus extérieur (eau, agents oxydants tels que l'air, lumière et/ou température) sont instables et subissent par exposition à un tel stimulus extérieur, une dégradation par un mécanisme d'hydrolyse chimique, d'oxydation, de photolyse ou de photodégradation, ou encore d'échange d'ions, ce qui conduit à des compositions cosmétiques qui deviennent peu efficaces et/ou sont d'aspect, d'odeur et/ou toucher inacceptables pour le consommateur. Il en est ainsi par exemple de l'acide ascorbique qui, du fait de cette dégradation, a tendance à jaunir les compositions le contenant. Le fait de mettre ces composés sensibles dans un film anhydre et de ne les incorporer dans la composition aqueuse qu'au moment de l'utilisation permet d'éviter tous ces problèmes.

Outre l'acide ascorbique, on peut citer comme composés sensibles à un stimulus extérieur, le rétinol (vitamine A) et ses dérivés ; l'urée ; la DHA (dihydroxyacétone) ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène) ; les acides gras polyinsaturés tels que l'acide gamma-linolénique.

Les composés utilisables dans le kit selon l'invention peuvent être notamment choisis parmi les actifs hydratants, les agents anti-séborrhéiques, les actifs anti-vieillissement, les actifs antimicrobiens, les actifs anti-inflammatoires ou apaisants, les actifs lipolytiques ou amincissants, les charges, les filtres solaires, les agents de coloration de la peau ou des cheveux, les actifs anti-cernes, les actifs anti-transpirants, les actifs déodorants, les actifs des traitements capillaires, les agents dépilatoires, les pigments, les colorants, les polymères, les parfums, les électrolytes, les ajusteurs de pH, les conservateurs, et leurs mélanges.
I) les actifs hydratants, tels que par exemple le lactate de sodium ; les polyols, et en particulier la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; la vaseline ; l'acide N-lauroyl pyrrolidone carboxylique et ses sels ; les acides gras essentiels ; les huiles essentielles ; et leurs mélanges.
II) les agent anti-séborrhéiques, choisis par exemple parmi :
   - le soufre et les dérivés soufrés ;
   - les sels de zinc tels que le lactate, le gluconate, le pidolate, le carboxylate, le salicylate et/ou le cystéate de zinc ;
   - le chlorure de sélénium ;
   - la vitamine B6 ou pyridoxine ;
   - le mélange de capryloyl glycine, de sarcosine et d'extrait de cinnamomum zeylanicum commercialisé notamment par la société SEPPIC sous la dénomination commerciale Sepicontrol A5 ;
   - un extrait de Laminaria saccharina commercialisé notamment par la société SECMA sous la dénomination commerciale Phlorogine® ;
   - un extrait de Spiraea ulmaria commercialisé notamment par la société SILAB sous la dénomination commerciale Sebonormine® ;
   - des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
   - un extrait de Serenoa repens commercialisé notamment par la société EUROMED;
   - des extraits de plantes du genre Silybum ;
   - des extraits végétaux contenant des sapogénines et en particulier les extraits de Dioscorées riches en diosgénine ou hécogénine ;
   - des extraits d'Eugenia caryophyllata contenant de l'eugénol et du glucoside d'eugényle ;
   - et leurs mélanges.
III) les actifs anti-vieillissement qui peuvent être choisis parmi tous les actifs susceptibles de traiter ou de prévenir tout signe de vieillissement de la peau. Ils peuvent être choisis par exemple parmi les agents anti-radicaux libres, les agents kératolytiques, les vitamines, les agents anti-élastase et anti-collagénase, les protides, les dérivés d'acides gras, les stéroïdes, les oligo-éléments, les agents blanchissants, les extraits d'algues et de planctons, les enzymes et co-enzymes, les flavonoïdes, les céramides, les agents tenseurs, les agents myorelaxants, et leurs mélanges.
   1) Comme agents anti-radicaux libres et anti-oxydants, on peut citer notamment les dérivés de l'acide phosphonique tels que l'acide éthylène diamine tétra(méthylène phosphonique), l'acide hexaméthylène diamine tétra(méthylène phosphonique), l'acide diéthylène triamine penta(méthylènephosphonique), et leurs sels et en particulier leurs sels de sodium ; l'acide éthylène diamine tétracétique et ses sels tels que le sel de sodium ; la guanosine ; la superoxydismutase ; le tocophérol (vitamine E) et ses dérivés (acétate) ; l'éthoxyquine ; la lactoferrine ; la lactoperoxydase, et les dérivés nitroxydes ; les superoxyde dismutases ; le glutathion peroxydase ; les extraits végétaux à activité antiradicalaire tels que l'extrait aqueux de germe de blé commercialisé par la société Silab sous la référence Detoxiline ; le thé vert ; et leurs mélanges.
   2) Comme agents kératolytiques, on peut citer par exemple les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5-salicylique ; les α-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; et leurs mélanges.
   3) Comme vitamines, outre les vitamines A, E et C indiquées ci-dessus, on peut citer en particulier la vitamine B3 (ou vitamine PP ou niacinamide) et ses dérivés (nicotinate de tocophérol, esters d'alcool nicotinyle et d'acides carboxyliques, 2-chloronicotinamide, 6-méthylnicotinamide, 6-aminonicotinamide, N-méthylnicotinamide, N,N-diméthylnicotinamide, N-(hydroxyméthyl)-nicotinamide, imide d'acide quinolinique, nicotinanilide, N-benzylnicotinamide, N-éthylnicotinamide, nifenazone, nicotinaldéhyde, acide isonicotinique, acide méthylisonicotinique, thionicotinamide, nialamide, acide 2-mercaptonicotinique, nicomol et niaprazine) ; la vitamine B5 (ou panthénol ou alcool panthénylique ou 2,4-dihydroxy-N-(3-hydroxypropyl)-3,3diméthylbutanamide), sous ses différentes formes: D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale ; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant et notamment les huiles d'origine végétale en contenant, telles que par exemple l'huile de jojoba, et leurs mélanges.
   4) Comme agents anti-élastase, on peut citer notamment les dérivés peptidiques, et notamment les peptides de graines de légumineuses tels que ceux commercialisés par les Laboratoires Sériobiologiques de Nancy sous la référence Parelastyl ; les dérivés de N-acylamino-amides décrits dans la demande FR-A-2,180,033, comme par exemple le {2-[acétyl-(3-trifluorométhyl-phényl)-amino]-3-méthyl-butyrylamino} acétate d'éthyle et l'acide {2-[acétyl-(3-trifluorométhylphényl)-amino]-3-méthyl-butyryl-amino} acétique, et leurs mélanges.
   5) Comme agents anti-collagénase, on peut citer les inhibiteurs de métalloprotéase, tels que l'acide éthylènediamine (EDTA), la cystéine, et leurs mélanges.
   6) Comme protides, on peut citer par exemple les protéines de blé ou de soja, leurs hydrolysats, comme ceux commercialisés par la société Silab sous la référence Tensine, et leurs mélanges.
   7) Comme dérivés d'acide gras, on peut citer notamment les phospholipides polyinsaturés dont les phospholipides d'acide gras essentiels de poulpe, et leurs mélanges.
   8) Comme stéroïdes, on peut citer par exemple la DHEA ou déhydroépiandrostérone, ses précurseurs biologiques, ses métabolites, et leurs mélanges. Par "précurseurs biologiques" de la DHEA, on entend notamment la Δ5-prégnénolone, la 17α-hydroxy prégnénolone et le sulfate de 17α-hydroxy prégnénolone. Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA), la 7-céto-DHEA qui est elle-même un métabolite de la 7β-hydroxy DHEA et la benzoyl DHEA.
   9) Comme oligo-éléments, on peut citer par exemple le cuivre, le zinc, le sélénium, le fer, le magnésium, le manganèse, et leurs mélanges.
   10) Comme agents blanchissants ou dépigmentants, on peut citer par exemple l'acide kojique et ses dérivés ; l'hydroquinone et ses dérivés tels que l'arbutine et ses esters ; la vitamine C et ses dérivés tels que l'ascorbyle phosphate de magnésium ; les sels tels que le calcium D pantéthéine sulfonate ; l'acide ellagique et ses dérivés ; le rucinol ; l'acide linoléique et ses dérivés ; les extraits de plantes, et notamment de réglisse, de mûrier ou de scutellaire ; le glutathion et ses précurseurs ; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylaminocarbonyl-4-aminophénol ; et les mélanges de ces composés.
   11) Comme extraits d'algues, on peut citer les extraits d'algues rouges ou brunes, et par exemple l'extrait d'algues brunes de la famille des Laminaires, comme les extraits de l'espèce *Laminaria digitata,* et plus particulièrement celui vendu par la société CODIF sous la dénomination Phycosaccharides, qui est une solution concentrée d'un oligosaccharide comprenant l'enchaînement de deux acides uriques : acide mannuronique et l'acide guluronique.
   12) Comme extraits de planctons, on peut citer le plancton en dispersion aqueuse (nom CTFA : Vitreoscilla Ferment) commercialisé sous la dénomination MEXORYL SAH par la société Chimex.
   13) Comme enzymes, on peut utiliser toute enzyme d'origine animale, microbiologique (bactérienne, fongique ou virale) ou synthétique (obtenue par synthèse chimique ou biotechnologique), sous forme cristalline pure ou sous une forme diluée dans un diluant inerte. On peut citer par exemple les lipases, les protéases, les phospholipases, les laccases, les cellulases, les peroxydases notamment les lactoperoxydases, les catalases, les superoxyde dismutases, ou parmi des extraits végétaux contenant les enzymes précitées, et leurs mélanges. Elles peuvent être choisies par exemple parmi celle vendue sous la dénomination commerciale « Subtilisine SP 554 » par la société Novo Nordisk et celle vendue sous la dénomination commerciale « LYSOVEG LS » par la société Laboratoires Sérobiologiques de Nancy.
   14) Comme co-enzymes, on peut utiliser notamment l'ubiquinone ou coenzyme Q10 qui appartient à la famille des benzoquinones à chaîne alkylénée, le coenzyme R qui est la biotine (ou vitamine H), et leurs mélanges.
   15) Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines, les 2-arylbenzofuranes, et leurs mélanges. Les isoflavonoïdes peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique. Comme isoflavonoïdes d'origine naturelle, on peut citer la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.
   16) Comme céramides, on peut utiliser tout type de céramide, d'origine naturelle ou synthétique, par exemple de type II, de type III, de type IV, de type V ou de type VI, et leurs mélanges. On peut citer par exemple comme céramides, la N-oléoyldihydrosphingosine, la N-stéaroylphytosphingosine, le N-α-hydroxybéhénoyldihydrosphingosine, la N-α-hydroxypalmitoyldihydrosphingosine, la N-linoléoyldihydrosphingosine, la N-palmitoyldihydrosphingosine, la N-stéaroyldihydrosphingosine, la N-béhénoyldihydrosphingosine, et leurs mélanges.
   17) Comme agents tenseurs, on peut citer par exemple:
      - les polymères synthétiques ;
      - les polymères d'origine naturelle ;
      - les silicates mixtes ;
      - les microparticules de cire ;
      - les particules colloïdales de charges inorganiques.
         - Les polymères synthétiques utilisables en tant qu'agent tenseur peuvent être choisis parmi :
            - les polymères et copolymères de polyuréthanne ;
            - les polymères et copolymères acryliques ;
            - les polymères d'acide isophtalique sulfoné ;
            - les polymères siliconés greffés ;
            - les polymères hydrosolubles ou hydrodispersibles comprenant des unités hydrosolubles ou hydrodispersibles et des unités à LCST (Lower Critical Solution Temperature).
         - Les copolymères de polyuréthanne, les copolymères acryliques et les autres polymères synthétiques pouvant être utilisés comme agents tenseurs peuvent notamment être choisis parmi les polycondensats, les polymères hybrides et les réseaux de polymères interpénétrés (IPNs). Par "réseau de polymères interpénétrés", on entend un mélange de deux polymères enchevêtrés, obtenu par polymérisation et/ou réticulation simultanée de deux types de monomères, le mélange obtenu ayant une température de transition vitreuse unique. Des exemples d'IPNs convenant comme polymères tenseurs, ainsi que leur procédé de préparation, sont décrits par exemple dans les documents US-A-6,139,322 et US-A-6,465,001. De préférence, l'IPN comprend au moins un polymère polyacrylique et, plus préférentiellement, il comprend en outre au moins un polyuréthane ou un copolymère de fluorure de vinylidène et d'hexafluoropropylène. Selon une forme d'exécution préférée, l'IPN comprend un polymère polyuréthane et un polymère polyacrylique. De tels IPNs sont notamment ceux de la série Hybridur commercialisés par la société Air Products. Un IPN particulièrement préféré comme polymère tenseur se trouve sous la forme d'une dispersion aqueuse de particules ayant une taille moyenne en poids comprise entre 90 et 110 nm et une taille moyenne en nombre d'environ 80 nm. Cet IPN a de préférence une température de transition vitreuse, Tg, qui va d'environ -60°C à +100°C. Un IPN de ce type est notamment commercialisé par la société Air Products sous la dénomination commerciale Hybridur X-01602. Un autre IPN convenant à une utilisation dans la présente invention est référencé Hybridur X18693-21 ou Hybridur 875 polymer dispersion.
         - D'autres IPNs convenant comme polymères tenseurs comprennent les IPNs constitués du mélange d'un polyuréthane avec un copolymère de fluorure de vinylidène et d'hexafluoropropylène, notamment ceux préparés comme décrit dans le document US-A-5,349,003. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société Atofina.
         - Des exemples de polymères siliconés greffés sont indiqués dans le document EP-A-1,038,519, qui est incorporé ici par référence. Un exemple préféré de polymère siliconé greffé est le polysilicone-8 (nom CTFA) qui est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle. Un polymère de ce type est notamment disponible sous la dénomination commerciale VS 80 (à 10% dans l'eau) ou LO 21 (sous forme pulvérulente) auprès de la société 3M. Il s'agit d'un copolymère de polydiméthylsiloxane à groupements propylthio, d'acrylate de méthyle, de méthacrylate de méthyle et d'acide méthacrylique.
         - Les polymères synthétiques susmentionnés peuvent se présenter sous forme de latex. Comme latex approprié pouvant être utilisé en tant qu'agent tenseur, on peut citer notamment les dispersions de polyester-polyuréthane et de polyéther-polyuréthane, telles que celles commercialisées sous les dénominations Avalure UR410 et UR460 par la société Noveon, et sous les dénominations Neorez R974, Neorez R981, Neorez R970, ainsi que les dispersions de copolymère acrylique telles que celles commercialisées sous la dénomination Neocryl XK-90 par la société Avecia.
         - Enfin, des polymères synthétiques appropriés comme polymères tenseurs peuvent être des polymères hydrosolubles ou hydrodispersibles comprenant des unités hydrosolubles ou hydrodispersibles et comprenant des unités à LSCT, lesdites unités à LCST présentant, en particulier, une température de démixtion dans l'eau de 5 à 40°C à une concentration massique de 1%. Ce type de polymère est plus amplement décrit dans le document FR-A-2,819,429.
         - Les polymères d'origine naturelle utilisables en tant qu'agent tenseur peuvent être choisis parmi :
            - les protéines végétales et hydrolysats de protéines végétales ;
            - les polysaccharides d'origine végétale, éventuellement sous forme de microgels, tels que l'amidon ;
            - les latex d'origine végétale.
         - Comme exemples de protéines végétales et hydrolysats de protéines végétales utilisables comme agents tenseurs, on peut citer les protéines et hydrolysats de protéines de maïs, de seigle, de froment, de sarrasin, de sésame, d'épeautre, de pois, de fève, de lentille, de soja et de lupin.
         - Les polysaccharides utilisables comme agents tenseurs peuvent être choisis parmi les polysaccharides d'origine naturelle, capables de former des gels thermoréversibles ou réticulés ainsi que des solutions. On entend par "thermoréversible" le fait que l'état gel de ces solutions de polymère est obtenu de façon réversible, une fois la solution refroidie en-dessous de la température de gélification caractéristique du polysaccharide utilisé. Comme polysaccharides d'origine naturelle de ce type, on peut citer les carraghénanes et tout particulièrement le kappa-carraghénane et le iota-carraghénane ; les agars ; les gellanes ; les alginates ; les pectines ; les chitosanes et leurs dérivés ; les pullulanes et leurs dérivés. Ces polysaccharides tenseurs peuvent être présents sous forme de microgels tels que décrits dans le document FR-A-2,829,025.
         - Les polysaccharides peuvent être choisis aussi parmi l'amidon et ses dérivés. L'amidon peut être de toute origine : par exemple riz, maïs, pomme de terre, manioc, pois, froment, avoine, et il peut être naturel ou éventuellement modifié par un traitement de type réticulation, acétylation, oxydation. Il peut être éventuellement greffé. Comme amidon pouvant être utilisé comme agent tenseur, on peut citer par exemple celui commercialisé par la société Lambert-Rivière sous la dénomination Remi Dri.
         - Une autre classe d'agents tenseurs utilisables selon l'invention est constituée par les silicates mixtes. Par cette expression, on entend tous les silicates d'origine naturelle ou synthétique renfermant plusieurs types de cations choisis parmi les métaux alcalins (par exemple Na, Li, K) ou alcalino-terreux (par exemple Be, Mg, Ca) et les métaux de transition. On utilise de préférence des phyllosilicates, à savoir des silicates ayant une structure dans laquelle les tétraèdres SiO₄ sont organisés en feuillets entre lesquels se trouvent enfermés les cations métalliques. Une famille de silicates particulièrement préférée comme agents tenseurs est celle des laponites. Les laponites sont des silicates de magnésium, de lithium et de sodium ayant une structure en couches semblable à celle des montmorillonites. La laponite est la forme synthétique du minéral naturel appelé "hectorite". On peut utiliser par exemple la laponite commercialisée sous la dénomination Laponite XLS ou Laponite XLG par la société Rockwood.
         - Une autre classe encore d'agents tenseurs est constituée par les microparticules de cire. Il s'agit de particules ayant un diamètre généralement inférieur à 5 µm, ou mieux à 0,5 µm, et constituées essentiellement d'une cire ou d'un mélange de cires choisies par exemple parmi les cires de Carnauba, de Candelilla ou d'Alfa. Le point de fusion de la cire ou du mélange de cires est de préférence compris entre 50°C et 150°C.
         - En variante encore, on peut utiliser comme agent tenseur, des particules colloïdales de charges inorganiques. Par "particules colloïdales", on entend des particules colloïdales en dispersion dans un milieu aqueux, hydroalcoolique ou alcoolique, ayant un diamètre moyen en nombre compris entre 0,1 et 100 nm, de préférence entre 3 et 30 nm. Comme exemples de charges inorganiques, on peut citer la silice, l'oxyde de cérium, l'oxyde de zirconium, l'alumine, le carbonate de calcium, le sulfate de baryum, le sulfate de calcium, l'oxyde de zinc et le dioxyde de titane. Une charge inorganique particulièrement préférée est la silice. Des particules colloïdales de silice sont notamment disponibles sous forme de dispersion aqueuse de silice colloïdale auprès de la société catalysts & Chemicals sous les dénominations commerciales COSMO S-40 et COSMO S-50. on peut aussi utiliser des particules colloïdales composites silice-alumine, telles que celles commercialisées par la société Grace sous les noms de Ludox AM, Ludox HSA et Ludox TMA.
   18) Comme agents myorelaxants qui sont des agents de lissage des rides d'expression, on peut citer par exemple les sapogénines (voir demande EP-A-1,352,643) ; l'adénosine (voir demande FR-0214828); les antagonistes des récepteurs associés aux canaux calciques (voir demande FR-A-2,793,681), et en particulier le manganèse et ses sels (voir demande FR-A-2,809,005) et l'alvérine (voir demande FR-A-2,798,590) ; les agonistes des récepteurs associés aux canaux chlore, dont la glycine (voir demande EP-A-0704210) et certains extraits *d'Iris pallida* (voir demande FR-A-2,746,641).
IV) les actifs antimicrobiens ou antifongiques, notamment le 2,4,4'-trichloro-2'-hydroxy diphényl éther (ou triclosan), le 3,4,4'-trichlorobanilide (ou triclocarban), le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxydiphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le document WO-A-93/18743, le farnesol, les phytosphingosines, les dérivés du sélénium, le pyrithione zinc, les tétracyclines comme l'érythromycine, et les mélanges de ces composés.
V) les actifs anti-inflammatoires ou apaisants tels que les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stéaryl glycyrrhétinate, l'acide 3- stéaroyloxy glycyrrhétique) ; l'acide ursolique et ses sels ; l'acide oléanolique et ses sels ; l'acide bétulinique et ses sels ; les extraits de Paeonia suffruticosa, de lactiflora, de Laminaria saccharina, de camomille, du Pygeum, de Boswellia serrata, de Centipeda cunnighami, d'Helianthus annuus, de Linum usitatissimum, de Cola nitida, de clou de girofle, d'Epilobium Angustifolium, de Bacopa monieri ; les sels de l'acide salicylique et en particulier le salicylate de zinc ; les phycosaccharides de la société Codif ; l'huile de Canola ; le bisabolol ; l'allantoïne ; le Sépivital EPC (diesterphosphorique de vitamine E et C) de la société Seppic ; les huiles insaturées en oméga 3 telles que les huiles de rosier muscat, de cassis, d'Echium, de poisson ; la capryloyl glycine ; le Seppicalm VG (sodium palmitoylproline et nymphea alba) de la société Seppic ; les tocotrienols ; le piperonal , l'aloe vera ; les phytostérols.
VI) les actifs lipolytiques ou amincissants, c'est-à-dire ayant une activité favorable, directe ou indirecte, sur la diminution du tissu adipeux, tels que :
   - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ; ou les associations contenant des dérivés xanthiques, comme l'association de caféine et de silanol (dérivé méthylsilanetriol de caféine), et par exemple le produit commercialisé par la société Exsymol sous la dénomination caféisilane C ;ou bien les composés d'origine naturelle contenant des bases xanthiques et notamment de la caféine, tels que les extraits de thé, de café, de guarana, de maté, de cola *(Cola Nitida)* et notamment l'extrait sec de fruit de guarana *(Paulina sorbilis)* contenant 8 à 10 % de caféine ; l'éphédrine et ses dérivés qui peuvent notamment se retrouver à l'état naturel dans les plantes telles que le Ma Huang (Ephedra plant) ;
   - les extraits végétaux et les extraits d'origine marine, qui soit sont actifs sur les récepteurs à inhiber, tels que les β-2-bloqueurs, les NPY-bloqueurs (décrits dans le document EP-A-838217), soit inhibent la synthèse des récepteurs aux LDL ou VLDL, soit sont actifs pour stimuler les récepteurs β et les protéines G, conduisant à l'activation de l'adénylcyclase. Comme extraits végétaux de ce type, on peut citer par exemple :
      - le *Garcinia Cambogia,*
      - les extraits de *Bupleurum chinensis,*
      - les extraits de lierre grimpant (*Hedera Helix*), d'arnica (*Arnica Montana L*), de romarin (*Rosmarinus officinalis N*), de souci (*Calendula officinalis*), de sauge (*Salvia officinalis L*), de ginseng (*Panax ginseng*), de millepertuis (*Byperycum Perforatum*), de fragon *(Ruscus aculeatus L*), d'ulmaire (*Filipendula ulmaria L*), d'orthosiphon *(Orthosiphon Stamincus Benth),* de bouleau *(Betula alba),* de cécropia et d'arganier,
      - les extraits de ginkgo biloba,
      - les extraits de prêle,
      - les extraits d'escine,
      - les extraits de cangzhu,
      - les extraits de *chrysanthellum indicum,*
      - les extraits de dioscorés riches en diosgénine ou la diosgénine ou hécogénine pure et leurs dérivés.
      - les extraits des plantes du genre *Armeniacea, Atractylodis Platicodon, Sinom-menum, Pharbitidis, Flemingia,*
      - les extraits de *Coleus* tels que C. *Forskohlii, C. blumei, C. esquirolii, C. scutellaroïdes , C. xanthantus* et C. *Barbatus,* tel que l'extrait de racine de *Coleus Barbatus* contenant 60 % de forskoline,
      - les extraits de Ballote,
      - les extraits de *Guioa,* de *Davallia,* de *Terminalia,* de *Barringtonia,* de *Trema, d'Antirobia.*
   Comme extrait d'origine marine on peut citer :
   - les extraits d'algues ou de phytopancton, tels que le rhodystérol ou l'extrait de *Laminaria Digitata* commercialisé sous la dénomination PHYCOX75 par la société Secma, l'algue skeletonema décrite dans le document FR-A-2,782,921 ou les diatomées décrites dans le document FR-A-2,774,292.
VII) Les charges, et spécialement celles qui apportent des effets optiques, c'est-à-dire les charges capables de conférer après application sur la peau, les effets optiques suivants :
   - matité ou diminution de la brillance ;
   - effet Soft Focus : On entend par effet «soft focus» la diminution visible des ridules, des pores et des irrégularités du micro relief de la peau, diminution obtenue instantanément après application de la composition cosmétique sur une peau présentant des imperfections liées à un micro relief irrégulier ;
   - homogénéisation et éclaircissement du teint : On entend par "homogénéité du teint", la diminution visible des dyschromies et irrégularités pigmentaires de la peau, obtenue instantanément après application de la composition cosmétique sur une peau présentant ces imperfections.
   Les charges sont des particules solides, généralement blanches, insolubles dans le milieu de la composition.
   Parmi les charges à effet optique utilisables dans l'invention, on peut citer les charges minérales (dioxyde de titane amorphe ou cristallisé sous forme rutile et/ou anatase, oxyde de zinc, oxyde de fer, oxyde de cérium, silice, alumine, nitrure de bore, talc, séricite, mica, ...) enrobées ou non ainsi que les charges composites, les nacres, les argiles, l'amidon et ses dérivés, les dispersions aqueuses de styrène acrylique, les particules de résine de mélamine-formaldéhyde ou d'urée-formaldéhyde, les dispersions aqueuses de polytétrafluoroéthylène (PTFE), les microdispersions de cires, les copolymères vinylpyrrolidone/1-triacontène, les cires et résines de silicone, les particules d'organopolysiloxane, les microsphères de terpolymère expansé de chlorure de vinylidène, d'acrylonitrile et de méthacrylate, les particules de nylon, les microbilles de cellulose, les fibres, les particules hémisphériques creuses de silicone tells que celles commercialisée sous les dénominations NLK-500 et NLK-503 par la société Takemoto Oil and Fat.
VIII) les filtres solaires, qui peuvent être choisis parmi les filtres chimiques UVA et UVB ou les filtres physiques habituellement utilisables dans le domaine cosmétique.
   Comme filtres UVB, on peut citer par exemple :
   (1) les dérivés de l'acide salicylique, en particulier le salicylate d'homomenthyle et le salicylate d'octyle ;
   (2) les dérivés de l'acide cinnamique, en particulier le p-méthoxycinnamate de 2-éthylhexyle, commercialisé par la société Givaudan sous la dénomination Parsol MCX;
   (3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
   (4) les dérivés de l'acide p-aminobenzoïque ;
   (5) le 4-méthyl benzylidène camphre commercialisé par la société Merck sous la dénomination EUSOLEX 6300 ;
   (6) l'acide 2-phénylbenzimidazole 5-sulfonique commercialisé sous la dénomination EUSOLEX 232 par la société Merck ;
   (7) les dérivés de 1,3,5-triazine, en particulier :
      - la 2,4,6-tris[p-(2'-éthylhexyl-1'-oxycarbonyl)anilino]-1,3,5-triazine commercialisé par la société BASF sous la dénomination UVINUL T150, et
      - le dioctyl butamido triazone commercialisé par la société Sigma 3V sous la dénomination UVASORB HEB ;
   (8) les mélanges de ces filtres.
   Comme filtres UVA, on peut citer par exemple :
   (1) les dérivés de dibenzoylméthane, en particulier le 4-(tert.-butyl) 4'-méthoxy dibenzoylméthane commercialisé par la société Givaudan sous la dénomination PARSOL 1789 ;
   (2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
   (3) les dérivés de benzophénone, par exemple :
      - la 2,4-dihydroxybenzophénone (benzophénone-1) ;
      - la 2,2',4,4'-tétra-hydroxybenzophénone (benzophénone-2) ;
      - la 2-hydroxy-4-méthoxy-benzophénone (benzophénone-3), commercialisé sous la dénomination UVINUL M40 par la société BASF ;
      - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
      - la 2,2'-dihydroxy-4,4'-diméthoxy-benzophénone (benzophénone-6) ;
      - la 5-chloro-2-hydroxybenzophénone (benzophénone-7) ;
      - la 2,2'-dihydroxy-4-méthoxy-benzophénone (benzophénone-8) ;
      - le sel disodique du diacide 2,2'-dihydroxy-4,4'-diméthoxy-benzophénone-5,5'-disulfonique (benzophénone-9) ;
      - la 2-hydroxy-4-méthoxy-4'-méthyl-benzophénone (benzophénone-10) ;
      - la benzophénone-11 ;
      - la 2-hydroxy-4-(octyloxy)benzophénone (benzophénone-12).
   (4) les dérivés silanes ou les polyorganosiloxanes à groupement benzophénone ;
   (5) les anthranilates, en particulier l'anthranilate de menthyle commercialisé par la société Haarman & Reiner sous la dénomination NEO HELIOPAN MA ;
   (6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
   (7) les dérivés siliciés de benzimidazolyl-benzazoles N-substitués ou de benzofuranyl-benzazoles, et en particulier:
      - le 2-[1-[3-[1,3,3,3-tétraméthyl-1-[(triméthylsilyl)oxy]disiloxanyl]propyl]-1H-benzimidazol-2-yl]-benzoxazole ;
      - le 2-[1-[3-[1,3,3,3-tétraméthyl-1-[(triméthylsilyl)oxy]disiloxanyl]propyl]-1H-benzimidazol-2-yl]-benzothiazole ;
      - le 2-[1-(3-trimethylsilanyl-propyl)-1H-benzimidazol-2-yl]-benzoxazole ;
      - le 6-méthoxy-1,1'-bis-(3-triméthylsilanyl-propyl)-1H,1'H-[2,2']bibenzimidazolyl-benzoxazole ;
      - le 2-[1-(3-trimethylsilanyl-propyl)-1 H-benzimidazol-2-yl]-benzothiazole, qui sont décrits dans le document EP-A-1 028 120 ;
   (8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
   (9) Les silicones benzotriazoles, qui sont décrits notamment dans le document EP-A- 0392 883, en particulier le silicone benzotriazole de formule : 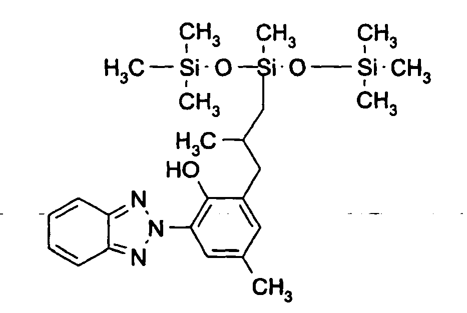
   (10) leurs mélanges.
   On peut aussi utiliser un mélange de plusieurs de ces filtres.
IX) les agents de coloration de la peau et des cheveux, en particulier les agents de la coloration de la peau tels que la dihydroxyacétone (DHA), les colorants naturels tels que les extraits végétaux comme par exemple les extraits de sorgho, et les azurants optiques.
   Les azurants optiques sont une famille de substances fluorescentes bien connues de l'homme du métier. De tels composés sont décrits dans "Fluorescent Whitening Agent, Encyclopedia of Chemical Technology, Kirk-Othmer", vol 11, p. 227-241, 4^{ème} édition, 1994, Wiley. Ce sont des agents de blanchiment de la peau par voie optique, constitués par des composés chimiques dotés de propriétés de fluorescence, qui absorbent dans l'ultraviolet (absorption maximale à une longueur d'onde inférieure à 400 nm) et réémettent de l'énergie par fluorescence pour une longueur d'onde comprise entre 380 nm et 830 nm. Une émission d'énergie comprise entre 400 nm et 480 nm résulte en une émission dans le bleu du domaine visible, ce qui contribue, lorsque cette émission a lieu sur la peau, à la blanchir visuellement.
   On peut plus particulièrement les définir comme des composés qui absorbent essentiellement dans l'UVA et l'UVB entre 290 et 400 nm et réémettent essentiellement entre 400 et 525 nm. Parmi les azurants optiques, on utilise de préférence des dérivés du stilbène, des dérivés coumariniques, des dérivés oxazole et benzoxazole, des dérivés imidazole. De tels composés sont facilement disponibles commercialement. Les azurants optiques préférentiellement utilisés dans le produit selon l'invention sont le di-styryl-4,4'bi-phenyl di-sulfonate commercialisés par la société Ciba Geigy sous le nom de Tinopal CBS-X ®. On peut aussi citer par exemple le 4,4'-bis[(4,6-diamilino-1,3,5-triazin-2-yl)amino]stilbène-2,2'-disulfonate de sodium, et le 2,5 thiophène di-yl bis(5 ter-butyl-1,3 benzoxazole) commercialisés par la société Ciba Geigy sous les noms de Tinopal SOP ® et d'Uvitex OB ®.
X) les actif anti-cernes tels que la vitamine K1 et ses dérivés, et les coumarines.
XI) les actifs anti-transpirants tels que les sels d'aluminium et/ou de zirconium, comme par exemple le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tétrachlorohydrate, l'aluminium zirconium trichlorohydrate et plus particulièrement l'aluminium chlorhydrate commercialisé par la société REHEIS sous la dénomination REACH 301 ou 303 ou par la société GUILINI CHEMIE sous la dénomination ALOXICOLL PF 40, et le sel d'aluminium et de zirconium commercialisé par la société REHEIS sous la dénomination REACH AZP-908-SUF ; les complexes d'hydroxychlorure de zirconium et d'hydroxychlorure d'aluminium avec un acide aminé tels que ceux décrits dans le document US-A-3,792,068, communément connus sous l'appellation "ZAG complexes", comme par exemple l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.
XII) Les actifs déodorants, tels que le pyrrolidone carboxylate de zinc (plus communément appelé pidolate de zinc), le sulfate de zinc, le chlorure de zinc, le lactate de zinc, le gluconate de zinc et le phénolsulfonate de zinc, le 2,4,4'-trichloro-2'-hydroxydiphényléther (Triclosan), le 2,4-dichloro-2'-hydroxydiphényléther, le 3',4',5'-trichlorosalicylanilide, le 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol, le monolaurate de glycérol ; les sels de polyhexaméthylène biguanide.
XIII) Les actifs des traitements capillaires, tels que (1) les agents inhibiteurs de la chute du cheveu ainsi que les agents stimulateurs de la pousse du cheveu, tels que le minoxidil, la biotine, l'aminexil, la cystéine, le finastèride, le 2,4 dipirymidine N-oxyde, le panthénol et dérivés, la flavanone T, les antagonistes de calcium comme le diltiazem, le vérapamil, l'alvérine, et la nifédipine, les hormones comme la progestérone, les agonistes du récepteur FP comme le latanoprost, les inhibiteurs de la 15-hydroxy prostaglandine déshydrogénase de type 1, les prostaglandines et leurs dérivés, ou plus généralement tout extrait végétal possédant une activité anti-5-alpha réductase de type I ou II ; (2) les agents antipelliculaires tels que la pyrithione de zinc, les dérivés de 1-hydroxy-2-pyrrolidone ou encore les sulfures de sélénium.
XIV) Les agents dépilatoires qui sont utilisés pour inhiber la pousse des poils, tels que l'acide thioglycolique et ses dérivés ; l'acide dithioglycolique et ses dérivés ; les sérines protéases décrites dans le document US-A-6,407,056 ; l'acide caféique ; la quercétine ; le gallate de propyle ; l'acide norhydroguaiarétique ou NDGA ; l'indométhacine ; l'hydrochlorure d'eflornithine ; les extraits végétaux tels que décrits dans le document US-A-6,171,595, comme les extraits de clou de girofle, de bouton de rose, de pimprenelle (burnet), de gambir ; les composés décrits dans le document US-A-6,075,052 ; le tétramisole ; l'orthovanadate de sodium ; le levamisole ; le chromoglycate disodique ; le nitrate de vanadium et le nitrate de gallium tel que décrit dans le document US-A-6,020,006 ; les composés décrits dans les documents US-A-4,885,289, US-A-4,720,489, US-A-5,132,293, US-A-5,096,911, US-A-5,095,007, US-A-5,143,925, US-A-5,328,686, US-A-5,440,090, US-A-5,364,885, US-A-5,411,991, US-A-5,648,394, US-A-5,468,476, US-A-5,475,763, US-A-5,455,608, US-A-5,674,477, US-A-5,728,736 et US-A-5,652,273, WO-A-94/27586, WO-A-94/27563 et WO-A-98/03149 ; les extraits de genévrier tels que décrits dans le document US-A-6,375,948.
XV) Les pigments qui sont utilisés notamment quand le produit obtenu est destiné au maquillage de la peau ou, quand il s'agit de pigment d'oxydes métalliques, quand le produit obtenu est destiné à constituer un produit solaire pour protéger la peau ou les cheveux colorés des rayons du soleil.
   Ces pigments peuvent être minéraux et/ou organiques, interférentiels, goniochromatiques, fluorescents, blancs, colorés, nacrés ou réfléchissants ou sous forme de paillettes. Par pigment, il faut comprendre des particules insolubles dans le milieu physiologique de la composition.
   On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille.
   Les pigments nacrés ou nacres peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Parmi les nacres disponibles sur le marché, on peut citer les nacres commercialisées sous les dénominations TIMICA® et FLAMENCO® par la société Engelhard et les nacres commercialisées sous la dénomination TIMIRON® par la société Merck.
   On peut aussi utiliser des pigments goniochromatiques, comme les pigments à structure multicouche interférentielle par exemple de structure Al/SiO₂/Al/SiO₂/Al, commercialisés par la société Dupont de Nemours ; de structure Cr/MgF₂/Al/MgF₂/Cr commercialisés sous la dénomination CHROMAFLAIR® par la société Flex ; de structure MoS₂/SiO₂/Al/SiO₂/MoS₂, Fe₂O₃/SiO₂/Al/SiO₂/Fe₂O₃ ou Fe₂O₃/SiO₂/Fe₂O₃/SiO₂/Fe₂O₃ commercialisés sous la dénomination de SICOPEARL® par la société BASF ; de structure MoS₂/SiO₂/mica-oxyde/SiO₂/MoS₂, Fe₂O₃/SiO₂/mica-oxyde/SiO₂/Fe₂O₃, TiO₂/SiO₂/TiO₂ ou TiO₂/Al₂O₃/TiO₂, commercialisés sous la dénomination XIRONA® par la société Merck. On peut encore citer les pigments commercialisés sous la dénomination INFINITE COLORS® de la société Shiseido.
   On peut aussi utiliser des pigments réfléchissants, comme des particules à substrat de verre revêtu d'argent, en forme de plaquettes, telles que celles vendues par exemple sous la dénomination MICROGLASS METASHINE REFSX 2025 PS® par la société Toyal ; des particules à substrat de verre revêtu d'alliage nickel/chrome/molybdène, telles que celles vendues par exemple sous les dénominations CRYSTAL STAR GF 55®, GF 2525® par la société Toyal ; des pigments de marque REFLECKS®, commercialisés par la société Engelhard, comportant un substrat de verre enrobé d'oxyde de fer brun ; des particules comportant un empilement d'au moins deux couches de polymères comme celles commercialisées par la société 3M sous la dénomination MIRROR GLITTER®.
   Comme particules goniochromatiques à cristaux liquides, on peut utiliser par exemple celles vendues par la société Chenix ainsi que celle commercialisée sous la dénomination HELICONE® HC par la société Wacker.
XVI) Les colorants qui vont apporter une couleur au(x) film(s) et/:ou à la composition aqueuse, notamment les colorants hydrosolubles tels que le sulfate de cuivre, le sulfate de fer, les sulfopolyesters hydrosolubles, les rhodamines, les colorants naturels comme le carotène et le jus de betterave, le bleu de méthylène, le caramel, le sel disodique de tartrazine et le sel disodique de fuschine, et leurs mélanges. On peut aussi éventuellement utiliser des colorants liposolubles. Le ou les colorants sont de préférence présents dans le ou les films. La composition aqueuse peut ainsi être colorée au moment de l'utilisation selon la teinte souhaitée à ce moment-là.
   Les colorants et les pigments permettent de moduler la couleur en fonction du but recherché (effet bonne mine, anti-cernes par exemple).
XVII) Les parfums qui peuvent être de tout type, soit des parfums composites comprenant un mélange de matières odoriférantes soit une matière odoriférante isolée. C'est ainsi que le kit peut comprendre plusieurs films comportant chacun une matière odoriférante différente de sorte que plusieurs films mélangés à la composition aqueuse donne un parfum particulier. On peut aussi introduire des parfums ayant des propriétés myorelaxantes, apportant un effet de détente lors de l'application du produit sur la peau.
   Les matières odoriférantes sont des composés usuellement utilisés par les parfumeurs et ils sont notamment décrits dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III. USA.
   Il peut s'agir de produits naturels (huiles essentielles, absolus, résinoïdes, résines, concrètes) et/ou synthétiques (hydrocarbures, alcools, aldéhydes, cétones, éthers, acides, esters, acétals, cétals, nitriles, saturés ou insaturés, aliphatiques ou cycliques).
   Des exemples d'huiles essentielles comprennent les huiles essentielles de citron, d'orange, d'anis, de bergamote, de rose, de géranium, de gingembre, de néroli, de basilic, de romarin, de cardamome, de camphre, de cèdre, de camomille, de santal, de sauge, et leurs mélanges, sans que cette liste soit limitative.
   Des exemples d'autres matières odoriférants sont notamment le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol, l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha -hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexène-carboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indane, les muscs-tétraline, les muscsisochromane, les cétones macrocycliques, les muscs-macrolactone, le brassylate d'éthylène et leurs mélanges.
XVIII) **Les** polymères qui, comme indiqué ci-dessus, peuvent être ceux intrinsèquement présents dans le ou les films anhydres, mais on peut utiliser aussi d'autres polymères présents dans des films anhydres et susceptibles d'apporter des propriétés rhéologiques particulières à la composition aqueuse. Comme polymères, on peut citer par exemple les polymères carboxyvinyliques modifiés ou non, tels que les produits commercialisés sous les dénominations Carbopol (nom INCl : carbomer) et Pemulen (nom INCl : Acrylates/C10-30 alkyl acrylate crosspolymer) par la société Noveon ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido-2-méthylpropane-sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom INCl : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom INCl : Polyacrylamide / C13-14 Isoparaffin / Laureth-7) et sous le nom de SIMULGEL 600 (nom INCl : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose et l'hydroxypropylcellulose ; les polymères associatifs comme les polyuréthannes associatifs, copolymères comportant au moins deux chaînes lipophiles hydrocarbonées ayant de 6 à 30 atomes de carbone, séparées par une séquence hydrophile, tels que les polyuréthannes commercialisés sous les dénominations SERAD FX1010, SERAD FX1100 et le SERAD FX1035 par la société HÜLS (nom INCl : Polyurethane), ceux commercialisés sous les dénominations Rheolate 255, Rheolate 278 et Rheolate 244 par la société RHEOX (nom INCl : Polyether-urea-polyurethane), ceux commercialisés sous les dénominations DW 1206F, DW 1206J, DW 1206B, DW 1206G par la société Röhm & Haas (nom INCl : Polyurethane), et celui commercialisé sous la dénomination Acrysol RM 2020 de la société Röhm & Haas. On peut aussi utiliser des polymères fixant tels que ceux classiquement utilisés dans les laques et les produits coiffants des cheveux.
XIX) Les électrolytes, et notamment les sels des métaux mono-, di- ou tri-valents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.
   Les ions constituant ces sels peuvent être choisis par exemple parmi les carbonates, les bicarbonates, les sulfates, les phosphates, les sulfonates, les glycérophosphates, les borates, les bromures, les chlorures, les nitrates, les acétates, les hydroxydes, les persulfates ainsi que les ions d'α-hydroxyacides (citrates, tartrates, lactates, malates) ou d'acides de fruits, les ions d'β-hydroxy-acides (salicylates, hydroxy-2 alcanoates, n-alkyl-salicylates et n-alcanoyl-salicylates), ou encore les ions d'acides aminés (aspartate, arginate, glycocholate, fumarate).
   On peut aussi utiliser un mélange de ces sels, y compris les mélanges naturels ou les mélanges dont la composition est proche de celle d'un mélange naturel, et en particulier d'un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange étant appelé "sels de la mer Morte" (« Dead Sea Bath Salts ») car il correspond aux principaux sels contenus dans la mer Morte.
   Les électrolytes préférés sont notamment le chlorure de sodium, le chlorure de magnésium et les sels de la mer Morte.
XX) Les ajusteurs de pH et notamment ceux habituellement utilisés dans le domaine cosmétique pour ajuster le pH des compositions à la valeur souhaitée. Il peut s'agir d'acides ou de bases, choisis parmi les bases inorganiques comme l"hydroxyde de sodium, les bases organiques comme les amines (triéthanolamine par exemple), les acides inorganiques comme l'acide chlorhydrique et les acides organiques comme l'acide citrique.
XXI) Les conservateurs et notamment tous ceux habituellement utilisés dans le domaine cosmétique, en particulier le phénoxyéthanol, les esters de l'acide parahydroxybenzoïque encore appelés Parabens comme le parahydroxybenzoate de méthyle (méthylparaben), le parahydroxybenzoate d'éthyle (éthylparaben) et le parahydroxybenzoate de propyle (propylparaben) et leurs mélanges ; Les libérateurs de formol comme l'imidazolidinyl urée ou la diazolidinyl urée ; les halogénoalkynylcarbamates comme le 3-iodo-2-propynyl butylcarbamate (IPBC) ; le caprylylglycol encore appelé 1,2-octanediol ; le benzoate de sodium ; le chlorure de N-(3-chloroallyl)-hexaminium (ou Quaternium-15) ; le chlorhydrate de polyhexaméthylène biguanide (nom CTFA : polyaminopropyl biguanide) ; les bromures d'alkyl-triméthylammonium comme le bromure de dodécyl-triméthylammonium, le bromure de myristyl-triméthylammonium, le bromure d'hexadécyltriméthylammonium, et leurs mélanges. Ces conservateurs peuvent être notamment présents dans la composition aqueuse.

Les composés cités ci-dessus peuvent être présents dans le produit final dans les quantités habituelles dans le domaine considéré, ces quantités dépendant du composé utilisé et du but recherché. Les composés peuvent être présents par exemple en une quantité allant de 0,001 à 20 % en poids, et mieux de 0,01 à 10 % en poids par rapport au poids de produit final. Selon le but recherché et le composé utilisé, les composés sont présents dans un ou plusieurs films ou dans la composition aqueuse.

Selon un mode particulier de réalisation de l'invention, au moins un film contient au moins un composé choisi parmi les composés sensibles à un stimulus extérieur, les agents kératolytiques, les charges, les agents tenseurs et leurs mélanges.

Le kit selon l'invention peut être utilisé notamment pour obtenir des produits destinés à être appliqués sur la peau, les muqueuses, les phanères ou les cheveux, notamment comme produits de soin de la peau ou comme produits de maquillage de la peau ou comme produits capillaires ou comme produits solaires.

Les exemples ci-après de compositions selon l'invention sont donnés à titre d'illustration et sans caractère limitatif. Les quantités y sont données en % en poids, sauf mention contraire, et les noms sont indiqués en nom chimique ou en nom CTFA selon les cas.

### Exemple 1 : Kit pour produit anti-rides

| Film anhydre | |
|---|---|
| Hydroxypropyl-méthylcellulose | 10 % |
| Glycérine | 5 % |
| Panthénol | 2 % |
| Adénosine | 0.15 % |
| Eau purifiée | 50 % |

Mode opératoire de préparation du film : L'ensemble des ingrédients est mélangé. Le mélange est déposé sur un papier siliconé à l'aide de techniques classiques d'enduction à rouleaux ou à racles, sous une épaisseur de l'ordre de 500 µm. Après séchage dans un tunnel à circulation d'air chaud à la température d'environ 50°C, le film obtenu est découpé à la forme désirée, par exemple sous forme de plaquettes carrées de 25 mm de côté.

| Composition aqueuse (serum) | |
|---|---|
| *Phase A* | |
| Gomme de xanthane | 0.2 % |
| Methyl Paraben | 0.2 % |
| Phenoxythanol | 0.35 % |
| Eau | qsq 100 % |
| | |

| *Phase B* | |
|---|---|
| Triéthanolamine | 0,2 % |
| Polyacryalamide / C13-C14 isoparaffine / laureth-7 (Sepigel 305) | 1 % |
| Diazolidinyl Yrea | 0,3 % |
| Glycérine | 7 % |

Mode opératoire de préparation du sérum : On chauffe à 75°C environ, sous agitation, la phase A, puis on verse la phase B dans la phase A. Ensuite, on arrête le chauffage tout en maintenant l'agitation jusqu'au retour à température ambiante. Une légère agitation est ensuite maintenue pendant 30 minutes.

Lors de l'utilisation, la consommatrice mélange dans le creux de sa main avec les doigts, une ou plusieurs plaquettes de film anhydre avec une dose comprise entre 100 et 500 mg de la composition aqueuse pendant une dizaine de secondes. Elle masse légèrement la surface à traiter de manière à favoriser l'étalement de la composition ainsi obtenue sur la peau et la pénétration des actifs qu'elle contient dans la peau. La composition obtenue peut être appliquée sur le visage ou le corps, notamment sur le visage pour le traitement des rides et ridules de la patte d'oie.

### Exemple 2 : Kit pour produit anti-âge

| Film anhydre | |
|---|---|
| Polyvinylpyrrolidone | 83 % |
| Acide ascorbique | 7 % |
| PEG-400 | 10 % |

Mode opératoire : L'ensemble des ingrédients est mélangé. Le mélange est déposé sur un papier siliconé à l'aide de techniques classiques d'enduction à rouleaux ou à racles, sous une épaisseur de l'ordre de 500 µm. Après séchage dans un tunnel à circulation d'air chaud à la température d'environ 50°C, le film obtenu est découpé à la forme désirée, par exemple sous forme de plaquettes carrées de 25 mm de côté.

| Composition aqueuse (émulsion H/E) | |
|---|---|
| *Phase A (phase aqueuse)* | |
| Gomme de xanthane | 0,25 % |
| Conservateur | 0,3 % |
| Eau | qsp 100 % |
| | |

| *Phase B (phase huileuse)* | |
|---|---|
| Beurre de karité | 2 % |
| Methyl glucose Sesquistearate | 2 % |
| PEG 20 methyl glucose Sesquistearate | 2 % |
| Huile d'amande d'abricot | 6 % |
| Alcool gras stéarylique | 2 % |
| | |

| *Phase C* | |
|---|---|
| Polyacrylamine / C13-14 isoparaffin / Laureth 7 (Sepigel 305) | 1 % |
| Conservateur | 0,35 % |
| Huile de silicone volatile | 10 % |

Mode opératoire : On chauffe à 75°C environ, sous agitation, la phase A, puis on verse sous très forte agitation, la phase B dans la phase A. Ensuite, on arrête le chauffage tout en maintenant l'agitation jusqu'au retour à température ambiante. On ajoute ensuite la phase C. Une légère agitation est ensuite maintenue pendant 30 minutes.

Lors de l'utilisation, la consommatrice mélange dans le creux de sa main avec les doigts, une ou plusieurs plaquettes de film anhydre avec une dose comprise entre 100 et 500 mg de la composition aqueuse, pendant une dizaine de secondes. Elle masse légèrement la surface à traiter de manière à favoriser l'étalement de la composition ainsi obtenue sur la peau, et la pénétration des actifs qu'elle contient dans la peau. La composition obtenue peut être appliquée sur le visage ou le corps notamment pour prévenir ou traiter les signes de l'âge.

### Exemple 3 : Kit de produit pour peaux grasses

| Film anhydre | |
|---|---|
| Hydroxypropylméthylcellulose | 68 % |
| Glycérine | 16 % |
| Silice (Sunsphere H33 de la société Asahi Glass) | 16 % |

Mode opératoire : L'ensemble des ingrédients est mélangé. Le mélange est déposé sur un papier siliconé à l'aide de techniques classiques d'enduction à rouleaux ou à racles, sous une épaisseur de l'ordre de 500 µm. Après séchage dans un tunnel à circulation d'air chaud à la température d'environ 50°C, le film obtenu est découpé à la forme désirée, par exemple sous forme de plaquettes carrées de 25 mm de côté.

| Composition aqueuse (Emulsion H/E) | |
|---|---|
| *Phase A (phase huileuse)* | |
| Stéarate de glycéryle | 1,35 % |
| Dimyristyl tartrate / cetearyl alcool /12-15 Pareth 7 / PPG 25 laureth 25 (Cosmacol PSE de la société Sasol) | 2 % |
| PEG-400 stéarate | 2,7 % |
| Isohexadécane | 2 % |
| Alcool stéarylique | 1,3 % |
| Vitamine E | 0,1 % |
| Conservateur | 0,6 % |
| | |

| *Phase B (phase aqueuse)* | |
|---|---|
| Eau | qsp 100 % |
| Hydroxyde de potassium | 1,5 % |
| Conservateur | 0,25 % |
| Ammonium polyacryloyldimethyltaurate (Hostacerin AMPS) | 0,5 % |
| | |

| *Phase C* | |
|---|---|
| Huile de silicone volatile | 6 % |

Mode opératoire : On chauffe à 75°C environ, sous agitation, la phase A et séparément la phase B, puis on verse la phase A dans la phase B sous très forte agitation. Ensuite, on arrête le chauffage tout en maintenant l'agitation jusqu'au retour à température ambiante. On ajoute la phase C. Une légère agitation est ensuite maintenue pendant 30 minutes.

Lors de l'utilisation, la consommatrice mélange dans le creux de sa main avec les doigts, un ou plusieurs plaquettes de films anhydres avec une dose comprise entre 100 et 500 mg de la composition aqueuse pendant une dizaine de secondes. Elle masse légèrement la surface à traiter de manière à favoriser l'étalement de la composition ainsi obtenue sur la peau, et la pénétration des actifs qu'elle contient dans la peau. La composition obtenue peut être appliquée notamment sur le visage où elle donne un effet matifiant immédiat.

### Exemple 4 : Crème de Soin

| Film anhydre | |
|---|---|
| Hydroxypropylméthylcellulose | 60 % |
| Glycérine | 30 % |
| Propylène glycol | 10 % |

Mode opératoire : L'ensemble des ingrédients est mélangé. Le mélange est déposé sur un papier siliconé à l'aide de techniques classiques d'enduction à rouleaux ou à racles, sous une épaisseur de l'ordre de 500 µm. Après séchage dans un tunnel à circulation d'air chaud à la température d'environ 50°C, le film obtenu est découpé à la forme désirée, par exemple sous forme de plaquettes carrées de 25 mm de côté.

| Composition aqueuse : | |
|---|---|
| *Phase A* | |
| Gomme de xanthane | 0,2 % |
| Methyl Paraben | 0,2 % |
| Phénoxyéthanol | 0,35 % |
| Eau | qsp 100% |
| | |

| *Phase B* | |
|---|---|
| Triéthanolamine | 0,2 % |
| Polyacrylamide / C13-C14 isoparaffine / laureth-7 (Sepigel 305) | 1 % |
| Diazolidinyl Urea | 0,3 % |
| Glycérine | 7 % |

Mode opératoire : On chauffe à 75°C environ, sous agitation, la phase A, puis on verse la phase B dans la phase A. Ensuite, on arrête le chauffage tout en maintenant l'agitation jusqu'au retour à température ambiante. Une légère agitation est ensuite maintenue pendant 30 minutes.

Lors de l'utilisation, la consommatrice mélange dans le creux de sa main avec les doigts, une ou plusieurs plaquettes de film anhydre avec une dose comprise entre 100 et 500 mg de la composition aqueuse pendant une dizaine de secondes. Elle masse légèrement la surface à traiter de manière à favoriser l'étalement de la composition ainsi obtenue sur la peau. La composition obtenue est une crème épaisse ayant de très bonnes propriétés d'application.

Cette crème peut être appliquée sur le visage ou sur le corps.

### Exemple 5: Crème de massage

| Film anhydre | |
|---|---|
| Hydroxypropylméthylcellulose | 40 % |
| Serad FX1100 | 20 % |
| Glycérine | 30 % |
| Propylène glycol | 10 % |

Mode opératoire : L'ensemble des ingrédients est mélangé. Le mélange est déposé sur un papier siliconé à l'aide de techniques classiques d'enduction à rouleaux ou à racles, sous une épaisseur de l'ordre de 500 µm. Après séchage dans un tunnel à circulation d'air chaud à la température d'environ 50°C, le film obtenu est découpé à la forme désirée, par exemple sous forme de plaquettes carrées de 25 mm de côté.

| Composition aqueuse : | |
|---|---|
| *Phase A* | |
| Gomme de xanthane | 0,2 % |
| Methyl Paraben | 0,2 % |
| Phenoxyéthanol | 0,35 % |
| Eau | qsp 100% |
| | |

| *Phase B* | |
|---|---|
| Triéthanolamine | 0,2 % |
| Polyacrylamide / C13-C14 isoparaffine / laureth-7 (Sepigel 305) | 1 % |
| Glycérine | 7 % |

Mode opératoire : On chauffe à 75°C environ, sous agitation, la phase A, puis on verse la phase B dans la phase A. Ensuite, on arrête le chauffage tout en maintenant l'agitation jusqu'au retour à température ambiante. Une légère agitation est ensuite maintenue pendant 30 minutes.

Lors de l'utilisation, la consommatrice mélange dans sa main avec les doigts, une ou plusieurs plaquettes de film anhydre avec une dose comprise entre 100 et 500 mg de la composition aqueuse pendant une dizaine de secondes. Elle masse légèrement la surface à traiter de manière à favoriser l'étalement de la composition ainsi obtenue sur la peau. La composition obtenue est une crème ayant les caractéristiques rhéologiques d'épaississement typique associé au polymère Serad FX. L'avantage est le contrôle des quantités appliquées et des qualités de massage.

Cette crème peut être appliquée sur le visage ou sur le corps pour un massage relaxant.

## Revendications

1. Kit de formulation d'un produit cosmétique comprenant (1) une composition aqueuse contenant au moins un corps gras, et (2) au moins un film anhydre hydrosoluble comportant au moins un composé qui est apte à modifier la composition aqueuse.

2. Kit de formulation d'un produit cosmétique comprenant :
i) une composition aqueuse ; et
ii) au moins un film anhydre hydrosoluble comportant au moins un composé qui, pour des raisons de stabilité, ne peut être mélangé à la composition que de façon extemporanée en vue de former ledit produit cosmétique.

3. Kit de formulation d'un produit cosmétique comprenant :
i) une composition aqueuse ;
ii) au moins un premier film anhydre hydrosoluble ; et
iii) au moins un second film anhydre hydrosoluble, le second film étant distinct du premier, par la concentration et/ou par la nature d'au moins un composé qu'il contient.

4. Kit de formulation customisée d'un produit cosmétique comprenant :
i) une composition aqueuse ;
ii) une pluralité de films anhydres hydrosolubles, identiques ou différents, destiné(s) à être mélangé(s) à la composition aqueuse pour former ledit produit cosmétique, et
iii) des instructions pour formuler à façon ledit produit cosmétique en fonction du nombre de films anhydres hydrosolubles identiques ou différents à mélanger à la composition.

5. Kit selon l'une quelconque des revendications 1 à 4, **caractérisé en ce que** le film est formé d'au moins un polymère hydrosoluble ou hydrodispersible

6. Kit selon la revendication précédente, **caractérisé en ce que** le polymère est choisi parmi les polymères de type protéique ; les polymères dérivant de la chitine ou du chitosane; les polymères polysaccharidiques ; les polymères ou copolymères acryliques ; les polymères vinyliques; les polymères d'origine naturelle ; et leurs mélanges.

7. Kit selon l'une quelconque des revendications précédentes, **caractérisé en ce que** le film a une épaisseur de 10 µm à 1000 µm et de préférence de 20 à 500 µm.

8. Kit selon l'une quelconque des revendications précédentes, **caractérisé en ce que** le film contient moins de 10 % d'eau.

9. Kit selon l'une quelconque des revendications 5 à 8, **caractérisé en ce que** la quantité de polymère va de 5 à 100 % en poids par rapport au poids total du film.

10. Kit selon l'une quelconque des revendications précédentes, **caractérisé en ce que** le film comprend un ou plusieurs plastifiants.

11. Kit selon l'une quelconque des revendications 2 à 10, **caractérisé en ce que** la composition aqueuse contient, outre l'eau, au moins un corps gras.

12. Kit selon l'une quelconque des revendications précédentes, **caractérisé en ce que** la composition aqueuse se présente sous forme d'émulsion comportant une phase aqueuse et une phase huileuse dispersées l'une dans l'autre.

13. Kit selon l'une quelconque des revendications précédentes, **caractérisé en ce que** le composé du ou des films est choisi parmi les actifs cosmétiques, les adjuvants de formulation et leurs mélanges.

14. Kit selon l'une quelconque des revendications précédentes, **caractérisé en ce que** la composition aqueuse contient un ou plusieurs composés choisis parmi les actifs cosmétiques, les adjuvants de formulation et leurs mélanges.

15. Kit selon l'une quelconque des revendications 13 ou 14, **caractérisé en ce que** les composés sont choisis parmi les actifs hydratants, les agents anti-séborrhéiques, les actifs anti-vieillissement, les actifs antimicrobiens, les actifs anti-inflammatoires ou apaisants, les actifs lipolytiques ou amincissants, les charges, les filtres solaires, les agents de coloration de la peau ou des cheveux, les actifs anti-cernes, les actifs anti-transpirants, les actifs déodorants, les actifs des traitements capillaires, les agents dépilatoires, les pigments, les colorants, les polymères, les parfums, les électrolytes, les ajusteurs de pH, les conservateurs, et leurs mélanges.

16. Kit selon l'une quelconque des revendications précédentes, **caractérisé en ce qu'**au moins un film contient au moins un composé choisi parmi les composés sensibles à l'eau, à l'oxydation, à la lumière et/ou à l'élévation de la température, les agents kératolytiques, les charges, les agents tenseurs et leurs mélanges.

17. Procédé de modification des caractéristiques de rhéologie, de couleur et/ou de parfum d'une composition aqueuse en vue de la formulation d'un produit cosmétique, consistant à ajouter à une composition aqueuse, un ou plusieurs films anhydres hydrosolubles contenant un ou plusieurs composés aptes à modifier la rhéologie, la couleur et/ou le parfum d'une composition aqueuse.

## Claims

1. Kit for formulating a cosmetic product, comprising (1) an aqueous composition containing at least one fatty substance and (2) at least one water-soluble anhydrous film comprising at least one compound that is capable of modifying the aqueous composition.

2. Kit for formulating a cosmetic product, comprising:
i) an aqueous composition; and
ii) at least one water-soluble anhydrous film comprising at least one compound which, for reasons of stability, can only be mixed with the composition extemporaneously in order to form the said cosmetic product.

3. Kit for formulating a cosmetic product, comprising:
i) an aqueous composition;
ii) at least one first water-soluble anhydrous film; and
iii) at least one second water-soluble anhydrous film, the second film being different from the first, with regard to the concentration and/or the nature of at least one compound it contains.

4. Kit for the customized formulation of a cosmetic product, comprising:
i) an aqueous composition;
ii) a plurality of identical or different water-soluble anhydrous films, intended to be mixed with the aqueous composition to form the said cosmetic product, and
iii) instructions for custom-formulating the said cosmetic product as a function of the number of identical or different water-soluble anhydrous films to be mixed with the composition.

5. Kit according to any one of Claims 1 to 4, **characterized in that** the film is formed from at least one water-soluble or water-dispersible polymer.

6. Kit according to the preceding claim, **characterized in that** the polymer is chosen from polymers of protein type; chitin-based or chitosan-based polymers; polysaccharide polymers; acrylic polymers or copolymers; vinyl polymers; polymers of natural origin; and mixtures thereof.

7. Kit according to any one of the preceding claims, **characterized in that** the film has a thickness of from 10 µm to 1000 µm and preferably from 20 to 500 µm.

8. Kit according to any one of the preceding claims, **characterized in that** the film contains less than 10% water.

9. Kit according to any one of Claims 5 to 8, **characterized in that** the amount of polymer ranges from 5% to 100% by weight relative to the total weight of the film.

10. Kit according to any one of the preceding claims, **characterized in that** the film comprises one or more plasticizers.

11. Kit according to any one of Claims 2 to 10, **characterized in that** the aqueous composition contains, besides water, at least one fatty substance.

12. Kit according to any one of the preceding claims, **characterized in that** the aqueous composition is in the form of an emulsion comprising an aqueous phase and an oily phase dispersed in each other.

13. Kit according to any one of the preceding claims, **characterized in that** the compound of the film(s) is chosen from cosmetic active agents and formulation adjuvants, and mixtures thereof.

14. Kit according to any one of the preceding claims, **characterized in that** the aqueous composition contains one or more compounds chosen from cosmetic active agents and formulation adjuvants, and mixtures thereof.

15. Kit according to either of Claims 13 and 14, **characterized in that** the compounds are chosen from moisturizing active agents, anti-seborrhoeic agents, anti-ageing active agents, antimicrobial active agents, anti-inflammatory or calmative active agents, lipolytic or slimming active agents, fillers, sunscreens, skin-colouring or hair-colouring agents, concealing active agents, antiperspirant active agents, deodorant active agents, hair-treating active agents, hair-removing agents, pigments, dyes, polymers, fragrances, electrolytes, pH adjusters and preserving agents, and mixtures thereof.

16. Kit according to any one of the preceding claims, **characterized in that** at least one film contains at least one compound chosen from compounds that are sensitive to water, to oxidation, to light and/or to an increase in temperature, keratolytic agents, fillers and tensioning agents, and mixtures thereof.

17. Process for modifying the rheology, colour and/or fragrance characteristics of an aqueous composition for the purpose of formulating a cosmetic product, which consists in adding to an aqueous composition one or more water-soluble anhydrous films containing one or more compounds capable of modifying the rheology, the colour and/or the fragrance of an aqueous composition.

## Patentansprüche

1. Kit für die Formulierung eines kosmetischen Produkts, der umfasst: (1) eine wässrige Zusammensetzung, die mindestens eine Fettsubstanz enthält, und (2) mindestens einen wasserlöslichen wasserfreien Film, der mindestens eine Verbindung enthält, die imstande ist, die wässrige Zusammensetzung zu modifizieren.

2. Kit für die Formulierung eines kosmetischen Produkts, der umfasst:
i) eine wässrige Zusammensetzung; und
ii) mindestens einen wasserlöslichen wasserfreien Film, der mindestens eine Verbindung enthält, die aus Stabilitätsgründen erst zum Zeitpunkt der Anwendung mit der Zusammensetzung vermischt werden darf, um das kosmetische Produkt zu bilden.

3. Kit für die Formulierung eines kosmetischen Produkts, der umfasst:
i) eine wässrige Zusammensetzung;
ii) mindestens einen ersten wasserlöslichen wasserfreien Film; und
iii) mindestens einen zweiten wasserlöslichen wasserfreien Film, wobei der zweite Film verschieden vom ersten Film ist durch die Konzentration und/ oder die Beschaffenheit mindestens einer Verbindung, die er enthält.

4. Kit für die kundenspezifische Formulierung eines kosmetischen Produkts, der umfasst:
i) eine wässrige Zusammensetzung;
ii) eine Vielzahl von wasserlöslichen wasserfreien Filmen, die identisch oder verschieden sind, der/die dafür vorgesehen ist/ sind, mit der wässrigen Zusammensetzung vermischt zu werden, um das kosmetische Produkt zu bilden, und
iii) Anweisungen, um das kosmetische Produkt maßgeschneidert in Abhängigkeit von der Zahl der identischen oder verschiedenen, mit der Zusammensetzung zu vermischenden wasserlöslichen wasserfreien Filme zu formulieren.

5. Kit nach einem der Ansprüche 1 bis 4, **dadurch gekennzeichnet, dass** der Film aus mindestens einem wasserlöslichen oder wasserdispergierbaren Polymer gebildet wird.

6. Kit nach dem vorhergehenden Anspruch, **dadurch gekennzeichnet, dass** das Polymer unter den Polymeren vom Proteintyp; den Polymeren, die von Chitin oder Chitosan abgeleitet werden; den Polysaccharidpolymeren; den Acrylpolymeren, oder -copolymeren; den Vinylpolymeren; den Polymeren natürlicher Herkunft; und deren Gemischen ausgewählt wird.

7. Kit nach einem der vorhergehenden Ansprüche, **dadurch gekennzeichnet, dass** der Film eine Dicke von 10 bis 1000 µm und vorzugsweise 20 bis 500 µm hat.

8. Kit nach einem der vorhergehenden Ansprüche, **dadurch gekennzeichnet, dass** der Film weniger als 10 % Wasser enthält.

9. Kit nach einem der Ansprüche 5 bis 8, **dadurch gekennzeichnet, dass** die Polymermenge im Bereich von 5 bis 100 Gew.-%, bezogen auf das Gesamtgewicht des Films, liegt,

10. Kit nach einem der vorhergehenden Ansprüche, **dadurch gekennzeichnet, dass** der Film einen oder mehrere Weichmacher enthält.

11. Kit nach einem der Ansprüche 2 bis 10, **dadurch gekennzeichnet, dass** die wässrige Zusammensetzung neben dem Wasser mindestens eine Fettsubstanz enthält.

12. Kit nach einem der vorhergehenden Ansprüche, **dadurch gekennzeichnet, dass** die wässrige Zusammensetzung in Form einer Emulsion vorliegt, die eine wässrige Phase und eine Ölphase umfasst, von denen die eine in der anderen dispergiert ist.

13. Kit nach einem der vorhergehenden Ansprüche, **dadurch gekennzeichnet, dass** die Verbindung des Films oder der Filme unter den kosmetischen Wirkstoffen, den Formulierungshilfsstoffen und deren Gemischen ausgewählt wird.

14. Kit nach einem der vorhergehenden Ansprüche, **dadurch gekennzeichnet, dass** die wässrige Zusammensetzung eine oder mehrere Verbindungen enthält, die unter den kosmetischen Wirkstoffen, den Formulierungshilfsmitteln und deren Gemischen ausgewählt werden,

15. Kit nach einem der Ansprüche 13 oder 14, **dadurch gekennzeichnet, dass** die Verbindungen ausgewählt werden unter den hydratisierenden Wirkstoffen, den Antiseborrhöika, den vor Alterung schützenden Wirkstoffen, den antimikrobiellen Mitteln, den entzündungshemmenden oder schmerzstill,enden Mitteln, den fettlösenden oder schlanker machenden Wirkstoffen, den Füllstoffen, den Sonnenschutzfiltern, den Mitteln zur Färbung der Haut oder der Haare, den Mitteln zur Verhinderung von Ringen um die Augen, den Antitranspirantien, den desodorierenden Wirkstoffen, den Mitteln zur Haarbehandlung, den Haarentfernungsmitteln, den Pigmenten, den Färbemitteln, den Polymeren, den Parfüms, den Elektrolyten, den Mitteln zur Einstellung des pH-Wertes, den Konservierungsmitteln und deren Gemischen.

16. Kit nach einem der vorhergehenden Ansprüche, **dadurch gekennzeichnet, dass** mindestens ein Film mindestens eine Verbindung enthält, die unter den Verbindungen, die gegenüber Wasser, Oxidation, Licht und/ oder Temperaturerhöhung empfindlich sind, den keratolytischen Mitteln, den Füllstoffen, den straffenden Wirkstoffen und deren Gemischen ausgewählt wird.

17. Verfahren zur Veränderung der rheologischen Eigenschaften, der Farbe und/oder des Geruchs einer wässrigen Zusammensetzung im Hinblick auf die Formulierung eines kosmetischen Produkts, das darin besteht, zu einer wässrigen Zusammensetzung einen oder mehrere wasserlösliche wasserfreie Filme zu geben, die eine oder mehrere Verbindungen enthalten, die imstande sind, die Rheologie, die Farbe und/oder den Geruch einer wässrigen Zusammensetzung zu verändern.
